(19) Europäisches Patentamt
European Patent Office
Office européen des brevets

(11) **EP 3 403 396 B1**

(12) **FASCICULE DE BREVET EUROPEEN**

(45) Date de publication et mention
de la délivrance du brevet:
**07.09.2022 Bulletin 2022/36**

(21) Numéro de dépôt: **17700230.0**

(22) Date de dépôt: **10.01.2017**

(51) Classification Internationale des Brevets (IPC):
***H04N 5/32*** *(2006.01)*

(52) Classification Coopérative des Brevets (CPC):
**H04N 5/32**

(86) Numéro de dépôt international:
**PCT/EP2017/050411**

(87) Numéro de publication internationale:
**WO 2017/121728 (20.07.2017 Gazette 2017/29)**

(54) **CIRCUIT DE DETECTION DE RAYONS X POUR CAPTEUR RADIOLOGIQUE DENTAIRE**

RÖNTGENSTRAHLENDETEKTIONSSCHALTUNG FÜR EINEN DENTALEN RADIOLOGISCHEN SENSOR

X-RAY DETECTION CIRCUIT FOR A DENTAL RADIOLOGY SENSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.01.2016 FR 1650211**

(43) Date de publication de la demande:
**21.11.2018 Bulletin 2018/47**

(73) Titulaire: **Teledyne E2V Semiconductors SAS**
**38120 Saint-Egrève (FR)**

(72) Inventeurs:
• **CHARRAT, Christine**
**38120 Saint-Egrève (FR)**
• **PAPAIX, Caroline**
**38950 Quaix-en-Chartreuse (FR)**
• **GESSET, Stéphane**
**38380 St.-Laurent-du-Pont (FR)**

(74) Mandataire: **Marks & Clerk France**
**Immeuble "Visium"**
**22, avenue Aristide Briand**
**94117 Arcueil Cedex (FR)**

(56) Documents cités:
EP-A2- 2 131 575      JP-A- S61 292 478
US-A- 5 887 049       US-A1- 2003 042 406
US-A1- 2006 208 161   US-A1- 2008 055 445
US-A1- 2015 346 361

**Description**

L'invention concerne le domaine de l'imagerie médicale, plus précisément pour des applications dentaires. Dans ce domaine, il existe des capteurs radiologiques dentaires qui sont constitués notamment de puces de capteurs d'image en technologie CMOS. Un capteur radiologique permet d'acquérir une image grâce à l'émission de rayons X par une source. Le capteur reçoit ces rayons X et les convertit en rayons visibles par une couche de matériau spécifique appelée scintillateur qui est collée sur le capteur d'image. L'image est obtenue par une lecture analogique des photons générés dans les photodiodes des pixels situés sous le scintillateur. Selon le type de photodiodes utilisées, les rayons X peuvent être convertis directement en un signal électrique sans passer par un scintillateur.

Afin de synchroniser l'émission des rayons X et le début de l'acquisition de l'image, un capteur radiologique doit nécessairement intégrer un système de détection de rayons X. Un tel système sert à détecter le début de l'émission des rayons X mais peut également servir à détecter la fin de l'acquisition de l'image.

L'invention porte plus particulièrement sur un circuit de détection de rayons X intégré dans un capteur radiologique dentaire.

Un circuit de détection de rayons X fonctionne globalement de la façon suivante. Il comporte principalement un ensemble de photodiodes et un circuit électronique de détection. Un flash de rayons X est émis par une source en direction des photodiodes qui convertissent les rayons X en courant. Le circuit de détection comprend principalement un amplificateur à transimpédance, un comparateur et un étage de sortie analogique. Le courant produit par l'ensemble des photodiodes est fourni à l'amplificateur qui produit un signal amplifié qui est ensuite comparé, via le comparateur, à un seuil afin de réaliser la détection.

Les recommandations des organismes normatifs médicaux imposent des contraintes à ce système. En effet, la dose de rayonnements reçue par les patients doit être limitée au strict minimum. Pour cela, deux solutions sont possibles, soit on réduit l'intensité du flux de rayons X émis, soit on réduit la durée d'exposition du patient, autrement dit la durée de détection. Dans les deux cas, il en résulte une diminution de l'amplitude du signal à détecter et donc une diminution du seuil de détection.

Or, la diminution de ce seuil engendre un autre problème. Dans les cabinets dentaires, un capteur radiologique est le plus souvent connecté directement à l'ordinateur personnel du dentiste, par exemple via un câble USB. La tension de référence fournie à l'amplificateur à transimpédance est générée à partir de tensions d'alimentation provenant de l'ordinateur du dentiste, donc d'une source externe au capteur. Ces tensions d'alimentation sont soumises à des variations aléatoires et sont également influencées par l'environnement électromagnétique. L'absence de tension de référence stable en entrée de l'amplificateur induit des variations sur le signal de sortie qui peuvent être telles que des dépassements du seuil de détection peuvent se produire de manière intempestive même en l'absence de rayons X reçus.

Il existe donc un problème à résoudre pour concevoir un circuit de détection de rayons X immune aux bruits présents sur les tensions d'alimentation, qui soit performant et fiable en termes de détection et qui permette une détection rapide avec un seuil de détection diminué. La demande de brevet américaine US 2008/0055445 A1 divulgue un capteur d'images matriciel comprenant des amplificateurs à transimpédance, leur tension' de référence est stable et immune aux bruits sur la tension d'alimentation pendant l'extraction des pixels de la matrice.

On connait différents circuits de détection de rayons X pour des capteurs radiologiques dentaires décrits notamment dans les brevets américains US 8119990, US8324587, US 6307915, US 5887049 et US 7592577. Les différentes solutions décrites dans ces documents ne permettent pas d'obtenir une détection rapide et fiable de rayons X pour des circuits utilisant une alimentation externe.

L'invention propose un circuit différentiel de détection de rayons X qui permet de s'affranchir des variations des tensions d'alimentation externes et qui, de ce fait, permet une diminution du seuil de détection sans augmenter la probabilité de fausse alarme liée à des déclenchements intempestifs du détecteur du fait de l'influence de bruits sur le signal amplifié.

L'invention a ainsi pour objet un circuit de détection de l'apparition de rayons X en vue de déclencher une prise d'image radiologique tel que défini à la revendication 1.

Selon un aspect particulier de l'invention, le circuit d'amplification comprend un premier interrupteur connecté entre la première entrée et la sortie de l'amplificateur.

Selon un aspect particulier de l'invention, le circuit d'amplification comprend un second interrupteur connecté entre la seconde entrée de l'amplificateur et la sortie de la source de tension.

Selon un aspect particulier de l'invention, le second interrupteur est configuré pour être en position fermée pendant la première partie de la phase d'initialisation pour charger la capacité avec une tension de référence générée par la source de tension.

Selon un aspect particulier de l'invention, le second interrupteur est configuré pour passer en position ouverte pendant la deuxième partie de la phase d'initialisation pour isoler la seconde entrée de l'amplificateur de la source de tension.

Selon un aspect particulier de l'invention, le premier interrupteur est configuré pour être en position fermée pendant la phase d'initialisation.

Selon un aspect particulier de l'invention, des moyens sont prévus pour ouvrir le premier interrupteur après la fin de la phase d'initialisation.

Selon un aspect particulier de l'invention, la capacité est réalisée en masquant une partie des photodiodes de détection et en connectant la sortie des photodiodes masquées à la seconde entrée de l'amplificateur.

Selon un aspect particulier de l'invention, les photodiodes masquées sont réparties uniformément au sein de l'ensemble de photodiodes de détection.

Selon un aspect particulier de l'invention, la proportion de photodiodes masquées par rapport aux photodiodes de détection est d'au moins 1/2.

L'invention a également pour objet un capteur d'image radiologique comprenant un circuit de détection de l'apparition de rayons X selon l'invention.

L'invention comprend également un procédé de détection de l'apparition de rayons X en vue de déclencher une prise d'image radiologique tel que défini par la revendication 12.

D'autres caractéristiques et avantages de la présente invention apparaîtront mieux à la lecture de la description qui suit en relation aux dessins annexés qui représentent :

- La figure 1a, un schéma synoptique général d'un circuit de détection de rayons X,
- La figure 1b, plusieurs chronogrammes illustrant le fonctionnement du circuit de détection,
- La figure 2, un schéma d'architecture d'un circuit amplificateur selon l'art antérieur,
- La figure 3, un schéma d'architecture d'un circuit de détection selon l'invention,
- La figure 4, un schéma d'une barrette de photodiodes d'un circuit de détection selon un mode de réalisation particulier de l'invention.

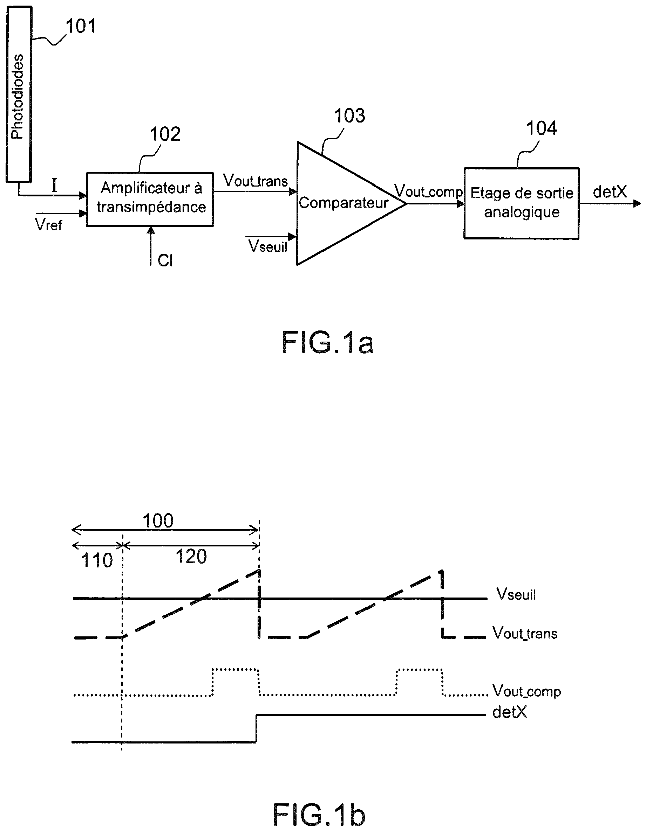

La figure 1a représente un schéma fonctionnel d'un circuit de détection de rayons X pour un capteur radiologique dentaire. La figure 1b illustre, sur plusieurs chronogrammes, le fonctionnement général du circuit.

Un circuit de détection de rayons X comprend principalement un ensemble 101 de photodiodes aptes à recevoir un flux de rayons X et à le convertir en un courant I qui est fourni sur une première entrée d'un amplificateur à transimpédance 102. Selon le type de photodiodes 101 utilisé, le courant I est généré directement ou indirectement par les photodiodes. Notamment, le courant I est généré indirectement lorsque le capteur radiologique utilise une couche de matériau appelée scintillateur qui converti, au préalable, les rayons X en rayons visibles.

Une alimentation en tension, provenant d'une source externe, est fournie sur une seconde entrée de l'amplificateur à transimpédance 102 qui est cadencé par une horloge CI. Le signal amplifié $V_{out\_trans}$ est ensuite transmis sur une première entrée d'un comparateur 103 qui reçoit sur une seconde entrée un signal seuil $V_{seuil}$. Le comparateur 103 produit en sortie un signal $V_{out\_comp}$ représentatif du dépassement du seuil $V_{seuil}$ par le signal amplifié $V_{out\_trans}$. Le signal de sortie du comparateur 103 est ensuite remis en forme via un étage de sortie analogique 104.

La figure 1b représente l'évolution des signaux d'entrée et de sortie du comparateur 103 ainsi que du signal de sortie de l'étage de sortie analogique 104. Les différents signaux correspondent à des signaux en tension exprimés en Volts.

Sur une période d'intégration 100, le signal amplifié $V_{out\_trans}$ est mis à une tension de référence $V_{ref}$ pendant une phase d'initialisation 110 puis il est amplifié pendant une phase d'intégration 120. La phase d'initialisation 110 sert à mettre l'entrée en tension de l'amplificateur à transimpédance 102 à une tension de référence $V_{ref}$ qui sert de point de référence pour l'intégration du courant. Cette phase est nécessaire pour stabiliser la tension de référence en entrée de l'amplificateur 102. La phase d'initialisation 110 a une durée sensiblement plus faible que la phase d'intégration 120. Par exemple, l'ordre de grandeur est une phase d'initialisation 110 de durée trois fois plus faible que la phase d'intégration 120.

Lorsque le signal amplifié $V_{out\_trans}$ dépasse le seuil $V_{seuil}$, le signal de sortie $V_{out\_comp}$ du comparateur 103 passe à une valeur haute. Le signal de sortie detX du circuit de détection passe ainsi à une valeur haute dès que le signal amplifié dépasse le seuil de détection afin de signaler la détection des rayons X. La valeur du seuil de détection est programmable.

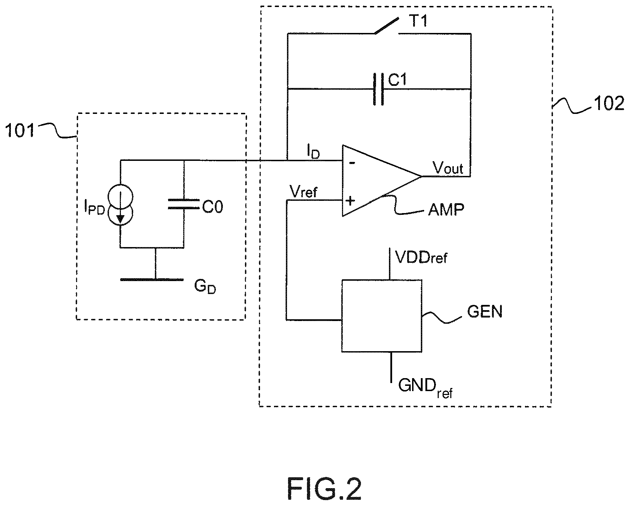

La figure 2 représente l'architecture d'un amplificateur à transimpédance 102 selon l'art antérieur connecté à un ensemble de photodiodes 101 modélisé sous la forme d'une source de courant $I_{PD}$ montée en parallèle avec une capacité parasite C0 et reliée à une masse $G_D$. La capacité parasite C0 modélise la somme des capacités parasites de toutes les photodiodes.

L'amplificateur à transimpédance 102 comprend un amplificateur AMP, un générateur GEN d'une tension de référence $V_{ref}$, une capacité $C_1$ et un interrupteur T1.

Le générateur GEN produit une tension de référence $V_{ref}$ qui vient alimenter une première entrée (l'entrée non inverseuse +) de l'amplificateur AMP. La capacité C1 est connectée entre la seconde entrée de l'amplificateur AMP à sa sortie et permet de régler le gain d'amplification du signal. L'interrupteur $T_1$ est également connecté entre la seconde entrée de l'amplificateur AMP à sa sortie. Le générateur GEN produit la tension de référence $V_{ref}$ à partir d'alimentations externes au circuit de détection. Les alimentations externes sont représentées sur la figure 2 sous la forme d'une alimentation externe positive $VDD_{ref}$ et d'une alimentation externe négative $GND_{ref}$. La seconde entrée de l'amplificateur à transimpédance (entrée inverseuse) est attaquée par le courant $I_D$ généré par l'ensemble 101 de photodiodes lorsqu'elles sont activées par un flux de rayons X.

Pendant la phase d'initialisation 110 (représentée sur les chronogrammes de la figure 1b), l'interrupteur T1 est en position fermée ce qui implique que la tension de sortie $V_{out}$ de l'amplificateur AMP est égale à la tension d'entrée $V_{ref}$ de référence. Pendant la phase d'intégration 120, l'interrupteur T1 est mis en position ouverte et l'amplificateur AMP amplifie alors le signal d'entrée. Sa fonction de transfert est donnée par la relation suivante, avec A le gain de l'amplificateur AMP :

$$Vout = \frac{1}{-\dfrac{C1-C0}{A}+C1}\int Idt + Vref = \frac{1}{-\dfrac{C1-C0}{A}+C1} I_{PD} \times \Delta t + Vref \qquad (1)$$

Le gain de l'amplificateur à transimpedance est fonction notamment de deux paramètres. Il dépend du choix de la fréquence d'échantillonnage qui joue sur le pas d'intégration $\Delta t$ mais aussi de la valeur de la capacité C1. Plus celle-ci est faible, plus le gain d'amplification est élevé.

Par ailleurs, les variations $DV_{ref}$ sur la tension d'entrée $V_{ref}$ et la tension de sortie $V_{out}$ sont également liées par la relation (2) :

$$Vout = (1+\frac{C0}{C1}) * DVref \qquad (2)$$

La relation (2) représente une fonction de transfert parasite qui vient s'ajouter à la fonction de transfert principale de l'amplificateur donnée par la relation (1). La somme des capacités parasites C0 présente une valeur significativement plus élevée que la capacité C1 qui est intégrée et dont la valeur doit être limitée afin de préserver le gain d'amplification. Typiquement l'ordre de grandeur de la somme des capacités parasites C0 est 100 pF tandis que l'ordre de grandeur de la capacité C1 est 50fF. Ainsi, le ratio C0/C1 est de l'ordre de 2000. Les variations de la tension d'entrée $V_{ref}$ sont donc amplifiées d'un facteur 2000 vers la tension de sortie $V_{out}$ de l'amplificateur AMP.

Comme indiqué en préambule, un problème existe lorsque la tension de référence $V_{ref}$ n'est pas stable, car les variations d'amplitude, mêmes faibles, sur cette tension induisent des variations amplifiées d'un facteur 2000 en sortie de l'amplificateur AMP. De telles variations peuvent ainsi induire des dépassements du seuil du comparateur 103 même en l'absence de rayons X, c'est-à-dire lorsque le courant $I_D$ est nul. Par exemple, une variation de 1mV sur la tension de référence lors de la phase d'intégration provoque une augmentation de 2 V sur la sortie de l'amplificateur AMP.

Or la tension de référence $V_{ref}$ est produite par un générateur GEN, intégré au dispositif de détection, mais qui utilise des alimentations externes. Typiquement, ces alimentations sont fournies par l'ordinateur personnel ou un équipement équivalent du dentiste qui vient connecter le capteur radiologique à cet équipement via, par exemple, une liaison USB ou tout autre moyen de connexion adapté.

Le fait d'utiliser des alimentations externes engendre potentiellement des phénomènes de variations et d'instabilité de la tension de référence $V_{ref}$ du fait de l'environnement électromagnétique aléatoire et de l'instabilité inhérente aux alimentations fournies par un ordinateur personnel qui ne sont pas destinées à un usage dédié pour un capteur à haute précision.

En outre, le problème réside également dans la valeur importante des capacités parasites C0 des photodiodes du fait que l'ensemble de photodiodes 101 est intégré à une matrice de grande dimension comprenant un grand nombre de photodiodes destinées à l'imagerie médicale. Ces capacités parasites sont inhérentes à la nature du dispositif et ne peuvent être éliminées.

Par ailleurs, il n'est pas possible non plus d'augmenter la capacité C1 de l'amplificateur pour compenser l'influence des capacités parasites C0 car cela entrainerait la réduction du gain d'amplification et donc la nécessité d'augmenter l'intensité du flux de rayons X ou d'augmenter la durée d'intégration afin d'obtenir un signal de sortie amplifié au même niveau. L'augmentation de l'exposition du patient aux rayons X va à l'encontre des recommandations des organismes normatifs médicaux et ne peut donc être une solution.

Pour corriger ce problème, l'invention propose d'éliminer la fonction de transfert parasite donnée par la relation (2) qui lie la tension de référence et la tension de sortie de l'amplificateur afin de ne conserver que la fonction d'amplification donnée par la relation (1). Pour cela, la solution consiste à générer une tension de référence $V_{ref}$ qui soit constante et qui ne subisse aucune variation, cet effet étant obtenu en rendant cette tension de référence indépendante d'alimentations externes au circuit de détection.

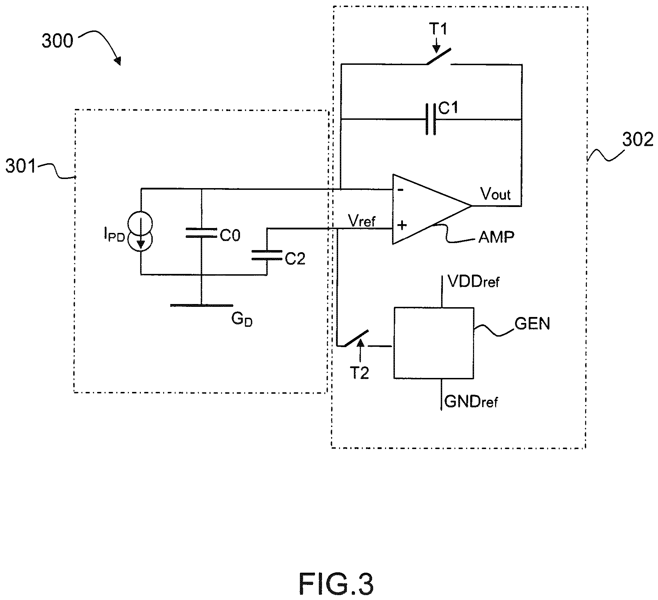

La figure 3 schématise l'architecture d'un détecteur de rayons X 300 selon l'invention. Sur cette figure, seuls sont représentés l'ensemble de photodiodes 301 ainsi que le circuit d'amplification 302. Le détecteur 103 et la chaine de sortie analogique 104 décrites à la figure 1a sont reprises à l'identique et ne sont donc pas représentés.

Le circuit d'amplification 302 selon l'invention comporte les mêmes éléments que le circuit d'amplification 102 selon l'art antérieur décrit à la figure 2 à la différence près qu'un second interrupteur $T_2$ est connecté entre la sortie du générateur GEN de tension de référence $V_{ref}$ et la première entrée de l'amplificateur AMP.

L'ensemble de photodiodes 301 est également modifié par rapport à l'ensemble 101 du circuit selon l'art antérieur décrit à la figure 2, par l'ajout d'une capacité de mémorisation C2 connectée entre la masse $G_D$ des photodiodes et la première entrée de l'amplificateur AMP. Cette capacité supplémentaire C2 peut être obtenue via une réalisation particulière utilisant une partie des photodiodes, cette réalisation étant décrite plus en détail plus loin dans la description. Comme explicité plus en détail ci après, il est important que la capacité supplémentaire C2 soit reliée à la même masse $G_D$ que les photodiodes produisant le courant I sur la seconde entrée de l'amplificateur AMP.

A la mise sous tension du circuit, on ferme le premier interrupteur $T_1$ et le second interrupteur $T_2$. Pendant une première partie de la phase d'initialisation, la tension de référence $V_{ref}$ est générée par le générateur GEN et la capacité C2, qui est reliée à la sortie du générateur GEN, est chargée avec la tension de référence $V_{ref}$.

Pendant une seconde partie de la phase d'initialisation, le second interrupteur T2 est ouvert. La tension de référence $V_{ref}$ est mémorisée par la capacité C2 et vient alimenter la première entrée de l'amplificateur AMP avec une tension de référence qui est stable et indépendante du générateur GEN. En effet, l'ouverture de l'interrupteur T2 permet d'isoler la première entrée de l'amplificateur AMP de l'environnement extérieur au circuit. La durée de la seconde partie de la phase d'initialisation est nécessaire pour garantir une stabilité de la tension de référence $V_{ref}$ en entrée de l'amplificateur AMP. Lorsque le second interrupteur T2 est ouvert, la tension de référence $V_{ref}$ est alors produite par la capacité supplémentaire C2 et lors de cette action, une impulsion parasite peut intervenir. Si on ouvre le premier interrupteur T1 simultanément au second interrupteur T2, l'impulsion parasite peut se propager vers la sortie de l'amplificateur et générer, à nouveau une variation d'amplitude parasite non désirée. C'est pour cette raison qu'un délai est nécessaire entre l'ouverture du second interrupteur T2 et l'ouverture du premier interrupteur T1, ce délai correspondant à la stabilisation de la tension de référence $V_{ref}$ suite à l'ouverture du second interrupteur T2. La durée de la seconde partie de la phase d'initialisation doit être minimisée pour éviter la diminution de la phase d'intégration.

La phase d'intégration est donc ensuite activée en ouvrant le premier interrupteur T1 pour activer la boucle de l'amplificateur via la capacité C1 et amplifier le courant produit par les photodiodes. Pendant la phase d'intégration, le second interrupteur T2 est maintenu ouvert.

Le fait que la capacité C2 supplémentaire introduite est connectée à la même masse $G_D$ que les photodiodes permet, lors de la phase d'intégration, également un renforcement de la stabilité du fait que les deux voies d'entrée de l'amplificateur AMP sont référencées à la même masse et ont les mêmes courants de fuite. Par exemple, si la capacité C2 était reliée à une masse extérieure au circuit, des variations potentielles de cette masse pourraient à nouveau engendrer des variations sur la tension de référence mémorisée dans la capacité. Le fait d'utiliser la même masse pour les photodiodes et pour la capacité supplémentaire C2 permet d'inhiber l'influence des variations de la masse car, si elles existent, elles impactent de façon symétrique à la fois le courant produit par les photodiodes et la tension de référence mémorisée dans la capacité C2.

Selon un mode de réalisation particulier de l'invention, la capacité C2 supplémentaire est réalisée en regroupant plusieurs photodiodes, initialement destinées à la détection, pour les masquer et utiliser uniquement leur capacité parasite en inhibant leur fonction de conversion de rayons X en courant. Le terme " masquer " est employé pour signifier qu'on rend une photodiode insensible aux rayons X en utilisant, par exemple du métal, pour couvrir la photodiode et empêcher les rayons X d'atteindre la photodiode. Les photodiodes masquées sont ainsi connectées à l'entrée en tension de l'amplificateur AMP tandis que les photodiodes non masquées sont connectées à l'entrée en courant de l'amplificateur AMP.

Le choix du nombre de photodiodes à masquer résulte d'un compromis. Plus le nombre de photodiodes masquées est important, plus la capacité C2 a une valeur importante et permettra de mémoriser la tension de référence sur une durée correspondant à la durée d'intégration. A l'inverse, un nombre important de photodiodes masquées diminue d'autant le nombre de photodiodes opérationnelles pour la détection des rayons X. Or, le nombre de photodiodes de détection influe directement sur l'intensité du courant produit qui ne doit pas être trop faible pour permettre une détection fiable.

Le masquage d'une partie des photodiodes dédiées à la détection est cependant possible notamment du fait qu'en réalisant le circuit de détection selon l'invention décrit à la figure 3, on élimine les détections intempestives en l'absence de courant et on peut ainsi diminuer la valeur du seuil $V_{seuil}$ de détection et donc s'adapter à un courant d'entrée d'intensité plus faible dû à un nombre de photodiodes de détection diminué.

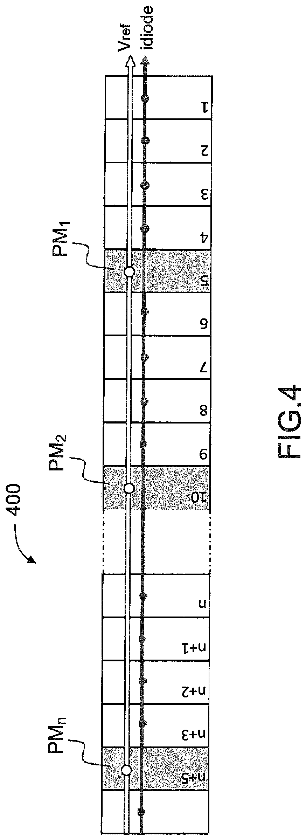

Selon un mode de réalisation particulier décrit à la figure 4, les photodiodes masquées sont avantageusement réparties uniformément au sein de l'ensemble des photodiodes afin de prendre en compte les dispersions technologiques entre les composants et d'avoir une meilleure homogénéité. Typiquement, un ensemble de photodiodes de détection est agencé dans une barrette 400 qui peut être positionnée sur une ligne ou une colonne de la matrice de pixels du capteur radiologique. Dans cette barrette 400, les photodiodes sont alignées et on vient masquer une photodiode sur n, avec n un entier supérieur ou égal à deux. Un bon compromis consiste, par exemple, à fixer la valeur de n à 5. Sur la figure 4, on a représenté un exemple de répartition de photodiodes masquées $PM_1, PM_2,.., PM_n$ avec un facteur n égal à 5.

L'invention n'est cependant pas limitée à l'agencement particulier décrit à la figure 4. En particulier, l'ensemble de photodiodes 101 peut être agencé de n'importe quelle autre manière, l'agencement choisi dépendant notamment de la conception du capteur radiologique. Le meilleur mode de fonctionnement de l'invention correspond, comme indiqué, à un positionnement des photodiodes masquées de façon uniforme au sein de l'ensemble de photodiodes, mais l'invention peut également s'étendre à un positionnement des photodiodes masquées non uniforme, par exemple un regroupement des photodiodes masquées sur une extrémité d'une barrette 400.

**Revendications**

1. Circuit de détection (300) de l'apparition de rayons X en vue de déclencher une prise d'image radiologique, comprenant un ensemble (301) de photodiodes relié à une masse ($G_D$), un circuit d'amplification à transimpédance (302) du courant fourni par l'ensemble des photodiodes et une capacité (C2), le circuit d'amplification à transimpédance (302) comprenant un amplificateur (AMP) et une source de tension (GEN) configurée pour produire une tension de référence ($V_{ref}$) à partir d'alimentations externes au circuit de détection, l'amplificateur (AMP) étant connecté, sur une première entrée de l'amplificateur (AMP), à une sortie de l'ensemble (301) de photodiodes, la capacité (C2) étant connectée entre la masse ($G_D$) et une seconde entrée de l'amplificateur (AMP), le circuit de détection (300) comprenant en outre un comparateur (103) pour comparer la tension de sortie de l'amplificateur (AMP) à une tension seuil, le circuit de détection (300) étant apte à produire un signal de déclenchement de prise d'image lorsque la tension de sortie de l'amplificateur (AMP) dépasse la tension seuil, le circuit de détection (300) étant **caractérisé en ce que** le circuit d'amplification à transimpédance (302) comprend :

   • Des moyens (T2, GEN) configurés pour charger la capacité (C2) avec la tension de référence ($V_{ref}$) générée par la source de tension (GEN) pendant une première partie d'une phase d'initialisation du circuit de détection, et pour isoler la seconde entrée de l'amplificateur (AMP) de la source de tension (GEN) pendant une deuxième partie de la phase d'initialisation du circuit de détection,
   • Une capacité (C1) reliée entre la première entrée de l'amplificateur (AMP) et une sortie de l'amplificateur pour intégrer le courant généré par l'ensemble (301) de photodiodes, pendant une phase de détection suivant la deuxième partie de la phase d'initialisation

2. Circuit de détection selon la revendication 1 dans lequel le circuit d'amplification (302) comprend un premier interrupteur (T1) connecté entre la première entrée et la sortie de l'amplificateur (AMP).

3. Circuit de détection selon l'une quelconque des revendications 1 ou 2 dans lequel moyens (T2, GEN) comprennent un second interrupteur (T2) connecté entre la seconde entrée de l'amplificateur (AMP) et la sortie de la source de tension (GEN).

4. Circuit de détection selon la revendication 3 dans lequel le second interrupteur (T2) est configuré pour être en position fermée pendant la première partie de la phase d'initialisation pour charger la capacité (C2) avec une tension de référence ($V_{ref}$) générée par la source de tension (GEN).

5. Circuit de détection selon la revendication 4 dans lequel le second interrupteur (T2) est activé en position ouverte pendant la deuxième partie de la phase d'initialisation pour isoler la seconde entrée de l'amplificateur (AMP) de la source de tension (GEN).

6. Circuit de détection selon l'une des revendications 4 ou 5 dans lequel le premier interrupteur (T1) est configuré pour être en position fermée pendant la phase d'initialisation.

7. Circuit de détection selon l'une des revendications 2 à 6 dans lequel il est prévu des moyens pour ouvrir le premier interrupteur (T1) après la fin de la phase d'initialisation.

8. Circuit de détection selon l'une quelconque des revendications précédentes dans lequel la capacité (C2) est réalisée en masquant une partie des photodiodes de détection et en connectant la sortie des photodiodes masquées à la seconde entrée de l'amplificateur.

9. Circuit de détection selon l'une des revendications précédentes dans lequel les photodiodes masquées sont réparties uniformément au sein de l'ensemble (301) de photodiodes de détection.

10. Circuit de détection selon la revendication 8 dans lequel la proportion de photodiodes masquées par rapport aux photodiodes de détection est de l'ordre de 1/n avec n, un entier au moins égal à deux.

11. Capteur d'image radiologique comprenant un circuit de détection (301) de l'apparition de rayons X en vue de déclencher une prise d'image radiologique selon l'une des revendications précédentes.

12. Procédé de détection de l'apparition de rayons X en vue de déclencher une prise d'image radiologique, mettant en œuvre le circuit de détection (300) selon l'une des revendications 3 à 10, **caractérisé en ce qu'**il comprend les phases suivantes :

- une phase d'initialisation du circuit de détection (300) comprenant :

o une première partie de phase d'initialisation comprenant la fermeture du second interrupteur (T2) pour charger la capacité (C2) avec la tension de référence (Vref) générée par la source de tension (GEN), le premier interrupteur (T1) étant en position fermée ;
o une deuxième partie de phase d'initialisation comprenant l'ouverture du second interrupteur (T2) pour isoler la seconde entrée de l'amplificateur (AMP) de la source de tension (GEN), le premier interrupteur (T1) étant en position fermée ;

- une phase de détection comprenant l'ouverture du premier interrupteur (T1), et l'intégration du courant généré par l'ensemble (301) de photodiodes par une capacité (C1) reliée entre la première entrée du circuit d'amplification à transimpédance (302) et la sortie de l'amplificateur (AMP), de façon à fournir une tension de sortie de l'amplificateur (AMP) ;
- une phase de comparaison de la tension de sortie de l'amplificateur (AMP) à une tension seuil ;
- une phase de fourniture d'un signal de déclenchement de prise d'image lorsque la tension de sortie de l'amplificateur (AMP) dépasse la tension seuil.

**Patentansprüche**

1. Schaltung (300) zum Detektieren des Auftretens von Röntgenstrahlen, um die Aufnahme eines Röntgenbildes auszulösen, umfassend einen Satz (301) von mit einer Masse (G_D) verbundenen Fotodioden, eine Transimpedanz-Verstärkerschaltung (302) für den von dem Fotodiodensatz gelieferten Strom und einen Kondensator (C2), wobei die Transimpedanz-Verstärkerschaltung (302) einen Verstärker (AMP) und eine Spannungsquelle (GEN) umfasst, konfiguriert zum Erzeugen einer Referenzspannung (V_ref) auf der Basis von Stromversorgungen außerhalb der Detektionsschaltung, wobei der Verstärker (AMP) an einem ersten Eingang des Verstärkers (AMP) mit einem Ausgang des Fotodiodensatzes (301) verbunden ist, wobei der Kondensator (C2) zwischen der Masse (G_D) und einem zweiten Eingang des Verstärkers (AMP) geschaltet ist, wobei die Detektionsschaltung (300) ferner einen Komparator (103) zum Vergleichen der Ausgangsspannung des Verstärkers (AMP) mit einer Schwellenspannung umfasst, wobei die Detektionsschaltung (300) ein Bildaufnahmeauslösesignal erzeugen kann, wenn die Ausgangsspannung des Verstärkers (AMP) die Schwellenspannung überschreitet, wobei die Detektionsschaltung (300) **dadurch gekennzeichnet ist, dass** die Transimpedanz-Verstärkerschaltung (302) Folgendes umfasst:

• Mittel (T2, GEN), die zum Laden des Kondensators (C2) mit der von der Spannungsquelle (GEN) erzeugten Referenzspannung (V_ref) während eines ersten Teils einer Initialisierungsphase der Detektionsschaltung und zum Isolieren des zweiten Eingangs des Verstärkers (AMP) von der Spannungsquelle (GEN) während eines zweiten Teils der Initialisierungsphase der Detektionsschaltung konfiguriert sind,
• einen Kondensator (C1), der zwischen dem ersten Eingang des Verstärkers (AMP) und einem Ausgang des Verstärkers geschaltet ist, um den vom Fotodiodensatz (301) erzeugten Strom während einer auf den zweiten Teil der Initialisierungsphase folgenden Detektionsphase zu integrieren.

2. Detektionsschaltung nach Anspruch 1, wobei die Verstärkerschaltung (302) einen ersten Schalter (T1) umfasst, der zwischen dem ersten Eingang und dem Ausgang des Verstärkers (AMP) geschaltet ist.

3. Detektionsschaltung nach Anspruch 1 oder 2, wobei die Mittel (T2, GEN) einen zweiten Schalter (T2) umfassen, der zwischen dem zweiten Eingang des Verstärkers (AMP) und dem Ausgang der Spannungsquelle (GEN) geschaltet ist.

4. Detektionsschaltung nach Anspruch 3, wobei der zweite Schalter (T2) so konfiguriert ist, dass er während des ersten Teils der Initialisierungsphase in der geschlossenen Position ist, um den Kondensator (C2) mit einer von der Spannungsquelle (GEN) erzeugten Referenzspannung ($V_{ref}$) zu laden.

5. Detektionsschaltung nach Anspruch 4, wobei der zweite Schalter (T2) während des zweiten Teils der Initialisierungsphase in der offenen Position aktiv ist, um den zweiten Eingang des Verstärkers (AMP) von der Spannungsquelle (GEN) zu isolieren.

6. Detektionsschaltung nach Anspruch 4 oder 5, wobei der erste Schalter (T1) so konfiguriert ist, dass er während der Initialisierungsphase in der geschlossenen Position ist.

7. Detektionsschaltung nach einem der Ansprüche 2 bis 6, wobei Mittel zum Öffnen des ersten Schalters (T1) nach Abschluss der Initialisierungsphase vorgesehen sind.

8. Detektionsschaltung nach einem der vorherigen Ansprüche, wobei der Kondensator (C2) durch Maskieren eines Teils der Detektionsphotodioden und durch Verbinden des Ausgangs der maskierten Photodioden mit dem zweiten Eingang des Verstärkers realisiert wird.

9. Detektionsschaltung nach einem der vorherigen Ansprüche, wobei die maskierten Photodioden gleichmäßig im Innern des Detektionsphotodiodensatzes (301) verteilt sind.

10. Detektionsschaltung nach Anspruch 8, wobei das Verhältnis von maskierten Fotodioden zu Detektionsfotodioden in der Größenordnung von 1/n liegt, wobei n eine ganze Zahl mindestens gleich zwei ist.

11. Röntgenbildsensor, der eine Schaltung (301) zum Detektieren des Auftretens von Röntgenstrahlen umfasst, um die Aufnahme eines Röntgenbildes nach einem der vorherigen Ansprüche auszulösen.

12. Verfahren zum Detektieren des Auftretens von Röntgenstrahlen zum Auslösen einer Röntgenbildaufnahme, das die Detektionsschaltung (300) nach einem der Ansprüche 3 bis 10 implementiert, **dadurch gekennzeichnet, dass** es die folgenden Phasen umfasst:

   - eine Initialisierungsphase der Detektionsschaltung (300), die Folgendes umfasst:

      o einen ersten Teil der Initialisierungsphase, der das Schließen des zweiten Schalters (T2) umfasst, um den Kondensator (C2) mit der von der Spannungsquelle (GEN) erzeugten Referenzspannung ($V_{ref}$) zu laden, wobei der erste Schalter (T1) in der geschlossenen Position ist;
      o einen zweiten Teil der Initialisierungsphase, der das Öffnen des zweiten Schalters (T2) umfasst, um den zweiten Eingang des Verstärkers (AMP) von der Spannungsquelle (GEN) zu isolieren, wobei der erste Schalter (T1) in der geschlossenen Position ist;

   - eine Detektionsphase, die das Öffnen des ersten Schalters (T1) und das Integrieren des von dem Fotodiodensatz (301) erzeugten Stroms durch einen Kondensator (C1) umfasst, der zwischen dem ersten Eingang der Transimpedanz-Verstärkerschaltung (302) und dem Ausgang des Verstärkers (AMP) geschaltet ist, um eine Ausgangsspannung des Verstärkers (AMP) zu liefern;
   - eine Phase des Vergleichens der Ausgangsspannung des Verstärkers (AMP) mit einer Schwellenspannung;
   - eine Phase des Lieferns eines Bildaufnahme-Auslösesignals, wenn die Ausgangsspannung des Verstärkers (AMP) die Schwellenspannung überschreitet.

**Claims**

1. A circuit (300) for detecting the appearance of X-rays with a view to triggering a radiological image capture, comprising a set (301) of photodiodes connected to a ground (GD), a transimpedance amplification circuit (302) of the current supplied by the set of photodiodes and a capacitor (C2), the transimpedance amplification circuit (302) comprising an amplifier (AMP) and a voltage source (GEN) configured to produce a reference voltage ($V_{ref}$) from power supplies external to the detection circuit, the amplifier (AMP) being connected, on a first input of the amplifier (AMP), to an output of the set (301) of photodiodes, the capacitance (C2) being connected between a ground ($G_D$) and a second input of the amplifier (AMP), the detecting circuit (300) further comprising a comparator (103) for comparing the output voltage of the amplifier (AMP) with a threshold voltage, the detection circuit (300) being able to produce an image capture trigger signal when the output voltage of the amplifier (AMP) exceeds the threshold voltage, the detection circuit (300) being **characterised in that** the transimpedance amplification circuit (302) comprises:

• means (T2, GEN) configured to charge the capacitor (C2) with the reference voltage ($V_{ref}$) generated by the voltage source (GEN) during a first portion of an initializing phase of the detecting circuit, and to isolate the second input of the amplifier (AMP) from the voltage source (GEN) during a second portion of the initializing phase of the detecting circuit,
• a capacitor (C1) connected between the first input of the amplifier (AMP) and an output of the amplifier to integrate the current generated by the assembly (301) of photodiodes, during a detecting phase following the second portion of the initializing phase.

2. The detecting circuit according to claim 1, wherein the amplifying circuit (302) comprises a first switch (T1) connected between the first input and the output of the amplifier (AMP).

3. The detecting circuit according to one of claims 1 or 2, wherein the means (T2, GEN) comprise a second switch (T2) connected between the second input of the amplifier (AMP) and the output of the voltage source (GEN).

4. The detecting circuit according to claim 3, wherein the second switch (T2) is configured to be in closed position during the first portion of the initializing phase in order to charge the capacitor (C2) with a reference voltage ($V_{ref}$) generated by the voltage source (GEN).

5. The detecting circuit according to claim 4, wherein the second switch (T2) is active in the open position during the second portion of the initializing phase in order to isolate the second input of the amplifier (AMP) from the voltage source (GEN).

6. The detecting circuit according to one of claims 4 or 5, wherein the first switch (T1) is configured to be in closed position during the initializing phase.

7. The detecting circuit according to one of claims 2 to 6, wherein means are provided for opening the first switch (T1) after the end of the initializing phase.

8. The detecting circuit according to one of the preceding claims, wherein the capacitor (C2) is produced by masking some of the detecting photodiodes and by connecting the output of the masked photodiodes to the second input of the amplifier.

9. The detecting circuit according to one of the preceding claims, wherein the masked photodiodes are uniformly distributed within the set (301) of detecting photodiodes.

10. The detecting circuit according to claim 8, wherein the proportion of masked photodiodes to the detecting photodiodes is of the order of 1/n with n being an integer at least equal to two.

11. X-ray image sensor comprising a circuit (301) for detecting the appearance of X-rays in order to trigger radiological image capture according to one of the preceding claims.

12. Method for detecting the appearance of X-rays with a view to triggering radiological image capture, implementing the detection circuit (300) according to one of claims 3 to 10, **characterised in that** it comprises the following phases:

- an initializing phase of the detection circuit (300) comprising:

° a first portion of the initializing phase comprising the closing of the second switch (T2) to charge the capacitor (C2) with the reference voltage ($V_{ref}$) generated by the voltage source (GEN), the first switch (T1) being in the closed position;

◦ a second portion of the initializing phase comprising the opening of the second switch (T2) to isolate the second input of the amplifier (AMP) from the voltage source (GEN), the first switch (T1) being in the closed position ;

- a detection phase comprising the opening of the first switch (T1), and the integration of the current generated by the assembly (301) of photodiodes by a capacitor (C1) connected between the first input of the transimpedance amplification circuit (302) and the output of the amplifier (AMP), so as to supply a voltage amplifier output (AMP);
- a phase for comparing the output voltage of the amplifier (AMP) with a threshold voltage;
- a phase for supplying an image capture trigger signal when the output voltage of the amplifier (AMP) exceeds the threshold voltage.

101

Photodiodes

I

Vref

102

Amplificateur à transimpédance

CI

Vout_trans

103

Comparateur

Vseuil

Vout_comp

104

Etage de sortie analogique

detX

## FIG.1a

100

110

120

Vseuil

Vout_trans

Vout_comp

detX

## FIG.1b

FIG.2

FIG.3

FIG.4

EP 3 403 396 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20080055445 A1
- US 8119990 B
- US 8324587 B
- US 6307915 B
- US 5887049 A
- US 7592577 B